# EUROPÄISCHE PATENTSCHRIFT

(11) **EP 2 789 311 B1**
(45) Veröffentlichungstag und Bekanntmachung des Hinweises auf die Patenterteilung: **12.08.2015**
(21) Anmeldenummer: 14153731.6
(22) Anmeldetag: 04.02.2014
(51) Int. Cl.: A61F 2/18

(54) **Gehörknöchelchenprothese mit aufgefüttertem Befestigungselement**
Auditory ossicle prosthesis with padded fastening element
Prothèse d'osselet avec élément de fixation rembourré

(30) Priorität: 08.04.2013 DE 102013103484
(43) Veröffentlichungstag der Anmeldung: 15.10.2014
(73) Patentinhaber: Heinz Kurz GmbH Medizintechnik, 72144 Dusslingen (DE)
(72) Erfinder: Steinhardt, Uwe, 72145 Hirrlingen (DE); Babu, Seilesh, Novi, MI, 48374 (US)
(74) Vertreter: Kohler Schmid Möbus Patentanwälte

(56) Entgegenhaltungen:
- EP-A1- 1 438 931
- DE-B3-102007 008 851

## Beschreibung

Die Erfindung betrifft eine aktive oder passive Gehörknöchelchenprothese, die zum Ersatz oder Überbrücken mindestens eines Elements der menschlichen Gehörknöchelchenkette ausgebildet ist, mit einem Schall übertragenden länglichen Prothesenkörper, welcher an seinem einen Ende ein erstes Ankoppelelement aufweist, das als eine Kopfplatte zur Anlage der Prothese am Trommelfell oder als ein Clip zur mechanischen Verbindung mit einem Glied der Gehörknöchelchenkette, insbesondere mit dem Ambossfortsatz oder mit dem Hammergriff, oder als ein Anschlussstück zur Schall leitenden Verbindung mit einem Aktor-Endstück eines aktiven Hör-Implantats gestaltet ist, wobei der Prothesenkörper an seinem anderen Ende ein zweites Ankoppelelement mit einer Zugangsöffnung in einen Aufnahmeraum aufweist, das für eine mechanische Verbindung der Prothese mit dem Steigbügel, insbesondere mit dem Steigbügel-Köpfchen (="caput"), als Glocke oder als Clip ausgebildet ist.

Im Bereich aktiver Hörimplantate sind solche Vorrichtungen etwa beschrieben in US 6,537,199 B1 oder beispielsweise in DE 10 2010 046 457 B3.

Bei passiven Gehörknöchelchenprothesen finden sich derartige Anordnungen mit unterschiedlich aufgebauten ersten und zweiten Befestigungselementen zum Beispiel in US-A 5,514,177, in WO 98/16175 A1, in EP 1 181 907 B1, in DE 10 2008 015 117 B3 oder etwa in DE 10 2009 016 468 B3.

Das menschliche Mittelohr mit seinen Gehörknöchelchen hat die Funktion, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. malleus), der Steigbügel (lat. stapes), der über seine Fußplatte (lat. basis stapedis) mit dem Innenohr verbunden ist, und der Amboss (lat. incus), der sich zwischen dem Hammer und dem Steigbügel befindet und mit diesen gelenkig verbunden ist. Beispielsweise die chronische Mittelohrentzündung ist eine Erkrankung des menschlichen Felsenbeins (= Knochen, in dem das gesamte Ohr sitzt), bei der es auf pathologisch-aggressive Art zu Abbauprozessen an der Gehörknöchelchenkette kommen kann. Dadurch wird das Schallsignal nicht oder nur unvollkommen zum Innenohr übertragen, was zur Schallleitungsschwerhörigkeit führt.

Hör-Implantate werden verwendet, um bei ganz oder teilweise fehlenden oder geschädigten Gehörknöchelchen des menschlichen Mittelohrs den auf die Ohrmuschel treffenden Schall bzw. ein entsprechendes Schallsignal zum Innenohr zu übertragen. Man unterscheidet dabei zwischen passiven Gehörknöchelchenprothesen einerseits, die Teile der Gehörknöchelchenkette physisch ersetzen, wobei die Schallleitung "passiv", also ohne Zuhilfenahme von energiebetriebenen Hilfsmitteln erfolgt, und aktiven Hör-Implantaten andererseits, die den Schallsignalen entsprechende energiebetriebene Signale aus einem meist elektronischen Verstärker eines extern oder auch intern angebrachten Hörgeräts mittels eines im Mittelohr implantierten Aktors empfangen, dort durch mechanische Bewegung wieder in akustische Schwingungen umsetzen und von einem vibrierenden Aktor-Endstück über ein geeignetes Verbindungselement zum Innenohr übertragen.

Passive Gehörknöchelchenprothesen dienen der Verbesserung der Schallübertragung bei unterschiedlichen pathologischen Befunden. Sie werden eingesetzt, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette und die Mittelohrmechanik mit ihren feinen Strukturen nur sehr eingeschränkt ersetzen können.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden am Amboss fixiert und ragen über einen Stempel (=Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte. Die vorliegende Erfindung beschäftigt sich ausschließlich mit Partial-Prothesen.

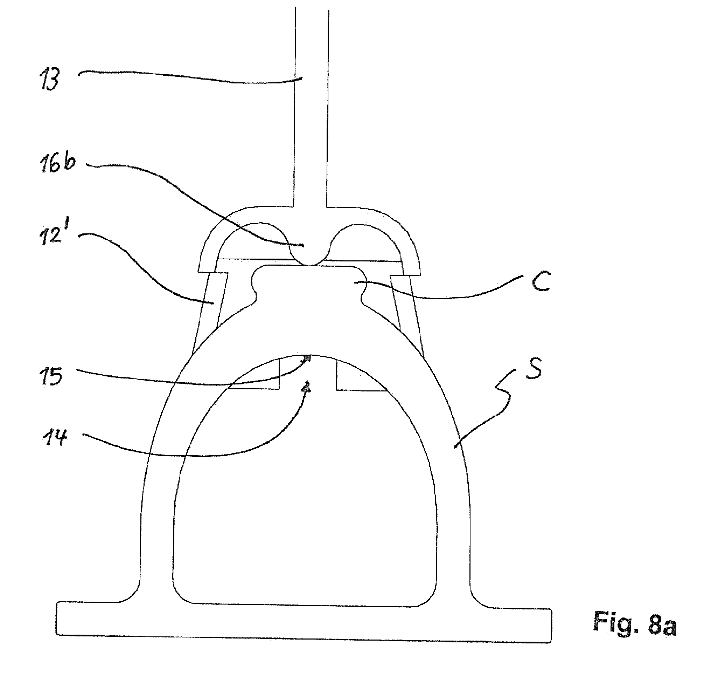

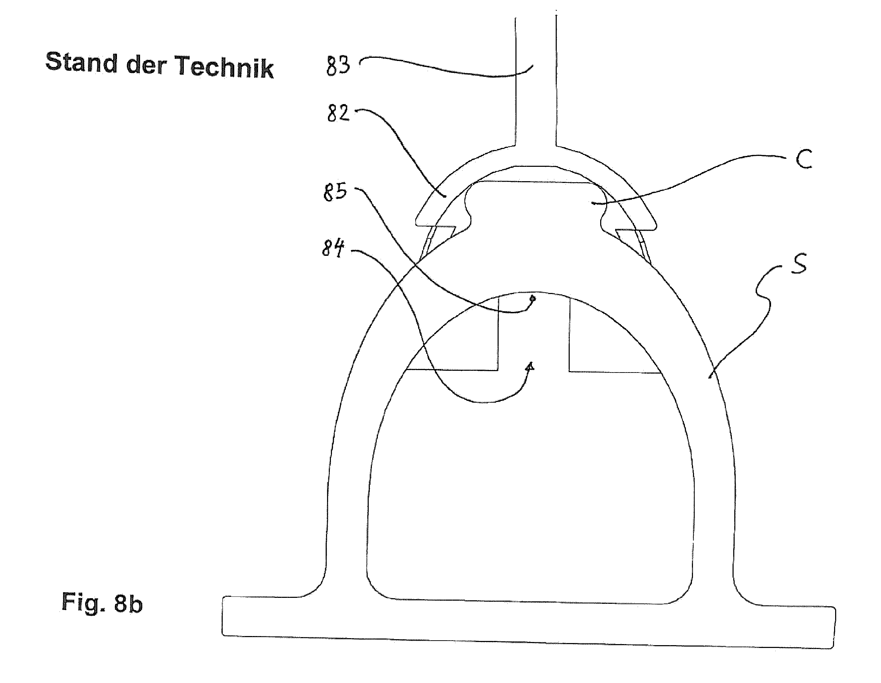

Wie aus den drei - stark vergrößerten - Aufnahmen von mehr oder weniger pathologischen menschlichen Steigbügel-Knochen in Fig. 8 deutlich wird, sind die anatomischen Unterschiede in der Gestalt und der absoluten Größe sowie auch in der jeweiligen Detail-Situation gerade im Bereich des Steigbügel-Köpfchens, wo ja die oben beschriebenen Partial-Prothesen mit ihrem zweiten Befestigungselement angekoppelt werden sollen, drastisch unterschiedlich. Eine wirklich optimale Ankopplung an dieser Position würde daher für jeden Patienten eine ganz individuelle Formgebung des jeweils verwendeten Ankoppelelements am Implantat erfordern, was natürlich mit einem Aufwand in vernünftigen Grenzen nicht zu leisten ist.

Hinzu kommt als nicht unbeachtliches Detailproblem, dass sich bei den gängigen Formen der bekannten und seit vielen Jahren eingesetzten Ankoppelelementen, etwa in Ausführungen als Stapes-Glocke oder auch bei Verwendung von Clips praktisch immer in Verlängerung des schaftförmigen Prothesenkörpers ein Hohlraum zwischen der Innenseite des Befestigungselements und dem oberen Bereich des Steigbügel-Köpfchens ausbildet, weil letzteres in der Regel eine eher abgeflacht Form aufweist, während die an dieser Stelle üblichen Ankoppelelemente konkav gewölbt sind.

Eine Gehörknöchelchenprothese, mit den Merkmalen des Oberbegriffs aus Anspruch 1, ist aus der EP-A-1438931 bekannt.

Aufgabe der vorliegenden Erfindung ist es demgegenüber, eine gattungsgemäße - aktive oder passive - Gehörknöchelchenprothese der eingangs beschriebenen Art möglichst kostengünstig und mit simplen technischen Mitteln dahin gehend zu verbessern, dass die oben beschriebenen Vorteile der bekannten Partial-Prothesen für die Handhabung im Bereich des Mittelohres sowie im Ergebnis die damit erzielbare Verbesserung der Schallleitung beibehalten bleiben, wobei jedoch nach der Implantation eine Hohlraumbildung in axialer Verlängerung des Prothesenkörpers zwischen der Innenseite des Befestigungselements und dem oberen Bereich des Steigbügel-Köpfchens sicher vermieden werden soll und wobei weitere Freiheitsgrade zur individuellen Anpassung an die anatomischen Besonderheiten des einzelnen Patienten hinsichtlich der Form, Größe und Lage seines Steigbügel-Knöchelchens eröffnet werden.

Erfindungsgemäß wird diese Aufgabe auf ebenso überraschend einfache wie wirkungsvolle Art und Weise dadurch gelöst, dass das zweite Ankoppelelement an der Innenfläche des Aufnahmeraums in axialer Fortsetzung des länglichen Prothesenkörpers einen vom Prothesenkörper weg in den Aufnahmeraum hinein ragenden Auffütterungsabschnitt aufweist, der im implantierten Zustand der Gehörknöchelchenprothese am Steigbügel, insbesondere am Steigbügel-Köpfchen anliegt und die Ausbildung eines Hohlraums zwischen dem Steigbügel und der Innenfläche des Aufnahmeraums in axialer Fortsetzung des länglichen Prothesenkörpers verhindert oder minimiert. Damit wird einerseits ein auch für lange Zeiträume verrutschsicherer und positionsstabiler Sitz der Gehörknöchelchenprothese am Steigbügel ermöglicht, andererseits wird auch die Schallleitung verbessert, weil kein Hohlraum als Schallhindernis mehr dazwischentritt.

Bei einer Klasse von vorteilhaften Ausführungsformen der erfindungsgemäßen Gehörknöchelchenprothese ist das zweite Ankoppelelement als seitlich mehrfach geschlitzte Glocke ausgeführt und mit der Glockenhaube an einem Ende des länglichen Prothesenkörpers befestigt. Die Schlitze an der Glocke haben den Vorteil, dass die Prothese auch dann über den Steigbügel gestülpt werden kann, wenn der oberste Teil des Steigbügels fehlen würde.

Eine Variante von Weiterbildungen dieser Ausführungsform zeichnet sich dadurch aus, dass die Glocke eine rund gewölbte Glockenhaube aufweist.

In vielen Fällen erweisen sich aber auch Weiterbildungen als günstig, bei welchen die Glocke eine abgeflachte und/oder von oben her eingedellte Glockenhaube aufweist. Dies hat zum Vorteil, dass es in axialer Verlängerung des Schaftes immer zu einem Kontakt kommt, so dass auch die Länge der Prothese eindeutig spezifiziert werden kann.

Eine alternative Klasse von Ausführungsformen zeichnet sich dadurch aus, dass das zweite Ankoppelelement als Clip mit mehreren, jeweils alternierenden seitlichen Zungen und Schlitzen ausgeführt ist. Diese Art von Ankoppelelementen wird häufig in Fällen eingesetzt, wo intraoperativ eine hohe Stabilität gefordert wird, so dass der Chirurg eine einfache Anwendung hinsichtlich der Technik auffindet.

Bei vielen Ausführungsformen der Erfindung wird in der Regel der Auffütterungsabschnitt kugelig oder ellipsoid gestaltet und symmetrisch zur verlängerten Achse des länglichen Prothesenkörpers angeordnet sein, so dass es immer zu einem direkten Kontakt zum Steigbügel kommt.

Für spezielle Situationen und individuelle Ausformungen des Steigbügel-Knöchelchens beim Patienten sind aber auch andere Ausgestaltungen der erfindungsgemäßen Gehörknöchelchenprothese vorteilhaft:
Eine weitere Ausführungsform etwa sieht vor, dass der Auffütterungsabschnitt kegelförmig gestaltet und symmetrisch zur verlängerten Achse des länglichen Prothesenkörpers angeordnet ist, wobei die Kegelspitze vom länglichen Prothesenkörpers weg in den Aufnahmeraum hinein ragt. Dies für zu einer sehr schallharten Verbindung, welche durch die punktartige Belastung seitlich fixiert wird.

Möglich sind aber auch Ausführungsformen, bei welchen der Auffütterungsabschnitt zylinderförmig gestaltet und symmetrisch zur verlängerten Achse des länglichen Prothesenkörpers angeordnet ist, wobei der Zylinder vom länglichen Prothesenkörpers weg in den Aufnahmeraum hinein ragt.

Bei weiteren Ausführungsformen ist der Auffütterungsabschnitt stempelförmig gestaltet und symmetrisch zur verlängerten Achse des länglichen Prothesenkörpers angeordnet, wobei ein den Stempelkörper tragender Stempelschaft vom länglichen Prothesenkörpers weg in den Aufnahmeraum hinein ragt.

Diese Ausführungsformen können, je nach den individuellen Bedürfnissen des Patienten, insbesondere der genauen Formgebung des Steigbügel-Köpfchens wiederum in unterschiedlicher Weise weitergebildet werden:
Bei einer Weiterbildung dieser Ausführungsformen weist der Stempelkörper eine in den Aufnahmeraum gerichtete konkave Auflagefläche auf, welche auch dann direkt greift, falls das Steigbügelköpfchen eine konvexe Form haben sollte.

Eine andere Weiterbildung zeichnet sich dadurch aus, dass der Stempelkörper eine in den Aufnahmeraum gerichtete ebene Auflagefläche aufweist.

In anders gelagerten Fällen kann aber eine Weiterbildung nützlich sein, bei der der Stempelkörper eine in den Aufnahmeraum gerichtete konvexe Auflagefläche aufweist, welche auch dann direkt greift, falls das Steigbügelköpfchen eine konkave Form haben sollte.

Um eine erhöhte Flexibilität bzw. Variabilität der Prothese zu erreichen, kann bei bevorzugten Ausführungsformen der Erfindung der längliche Prothesenkörper mindestens ein Gelenk, insbesondere ein Kugelgelenk aufweisen, was zum Vorteil hat, dass die Prothese auch hydrostatische Kräfte kompensieren kann

Vorteilhaft im Hinblick auf eine besonders hohe postoperative Beweglichkeit der Prothese sind Weiterbildungen, bei denen eine Vielzahl von aneinander angrenzenden weiteren Drehelementen vorgesehen ist, vorzugsweise eine Kugelgelenkkette, die Hindurchschieben der Kugelkette durch die Gelenk-Aufnahme und nachfolgendes Entfernen der überstehenden obersten Kugeln auf einfache Weise eine Längenvariabilität der Prothese ermöglicht.

Andere bevorzugte Weiterbildungen dieser Ausführungsformen sehen vor, dass das Kugelgelenk eine an seinem dem zweiten Ankoppelelement zugewandten Ende des Prothesenkörpers angebrachte Kugel, eine die Kugel auf ihrer vom zweiten Ankoppelelement abgewandten Seite bedeckende Hülle sowie eine Vertiefung in der dem zweiten Ankoppelelement zugewandten Seite umfasst, welche für die Kugel als Gelenkpfanne wirkt. Diese Ausführungsformen führen dazu, dass das Kugelgelenk einer weichen Lagerung unterliegt und damit Dämpfungen aufnimmt.

Besonders bevorzugte Varianten dieser Weiterbildungen zeichnen sich dadurch aus, dass die Hülle des in den Prothesenkörper integrierten Kugelgelenks aus einem Kunststoff-Verguss, vorzugsweise aus einem Silikon-Verguss gebildet ist.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein:
In vielen Ausführungsformen der Erfindung wird das erste Befestigungselement eine zur Anlage am Trommelfell ausgebildete Kopfplatte umfassen. Bei anderen Ausführungsformen kann beispielsweise die Prothese einerseits am Ambossfortsatz oder am Hammergriff befestigt sein. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Neben postoperativen Positionsverschiebungen ergibt sich nach der Implantation von Gehörknöchelchenprothesen häufig noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel.

Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen. Ein als Kopfplatte ausgeführtes erstes Befestigungselement der erfindungsgemäßen Gehörknöchelchenprothese sollte grundsätzlich eine wachstumsfördernde Beschichtung aufweisen.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Ganz besonders vorteilhaft sind Ausführungsformen der Erfindung, bei denen das zweite Ankoppelelement ganz oder teilweise aus Titan oder einem Material mit Formgedächtnis (=memory effect) und/oder mit superelastischen Eigenschaften, insbesondere aus Nitinol hergestellt ist. Die Verwendung derartiger Materialien ist auf dem Gebiet der Gehörknöchelchenprothesen an sich bekannt, erweist sich aber gerade im Zusammenhang mit der vorliegenden Erfindung als besonders wirkungsvoll.

Vorteilhaft im Hinblick auf die oben erwähnte postoperative Lageanpassung sind Ausführungsformen der Erfindung, bei denen die gesamte Prothese oder andere Teile davon, insbesondere auch das erste Ankoppelelement, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silikon, Polytetrafluorethylen (PTFE) oder Faserverbundwerkstoffen hergestellt sind. Mit diesen Materialien können postoperative Abstoßungsreaktionen in den meisten Fällen ebenfalls verhindert werden.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können jeweils einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1a: eine schematische räumliche Darstellung einer ersten Ausführungsform der erfindungsgemäßen Gehörknöchelchenprothese mit einer Trommelfell-Kopfplatte als erstem Befestigungselement und einem glockenförmigen zweiten Befestigungselement zur Auflage auf dem Steigbügel-Köpfchen sowie mit in den Aufnahmeraum des zweiten Befestigungselements hinein ragenden Auffütterungsabschnitt;
- Fig. 1b: eine Ausführungsform mit Kugelgelenk im Prothesenkörper;
- Fig. 1c: eine längenvariable Ausführungsform mit Kugelkette;
- Fig. 2a: eine Ausführungsform mit einem als Anschlussstück zur Schall leitenden Verbindung mit einem Aktor-Endstück eines aktiven Hör-Implantats ausgebildeten ersten Befestigungselement und mehrfach geschlitzter Glocke als zweiten Befestigungselement;
- Fig. 2b: Ausführungsform wie in Fig. 2a, jedoch mit clip-förmigem zweiten Befestigungselement mit mehreren, jeweils alternierenden seitlichen Zungen und Schlitzen;
- Fig. 3a: eine schematische Schnittdarstellung im Bereich eines als geschlitzte Glocke ausgeführten zweiten Befestigungselements mit halbkugelig gestaltetem Auffütterungsabschnitt;
- Fig. 3b: wie in Fig. 3a, aber mit kugelig gestaltetem Auffütterungsabschnitt;
- Fig. 4: wie in Fig. 3a, aber mit kegelig gestaltetem Auffütterungsabschnitt;
- Fig. 5: wie in Fig. 3a, aber mit zylinderförmig gestaltetem Auffütteru ngsa bsch nitt;
- Fig. 6a: wie in Fig. 3a, aber mit stempelförmig gestaltetem Auffütterungsabschnitt und einer in den Aufnahmeraum der Glocke gerichtete konkave Auflagefläche;
- Fig. 6b: wie in Fig. 6a, aber mit einer ebenen Auflagefläche;
- Fig. 6c: wie in Fig. 6a, aber mit einer konvexen Auflagefläche;
- Fig. 7: wie in Fig. 3a, aber mit in den Prothesenkörper endseitig integrierten Kugelgelenk, wobei der Auffütterungsabschnitt des zweiten Befestigungselements als Gelenkpfanne aus gebildet ist;
- Fig. 8a: schematische Schnittdarstellung einer Gehörknöchelchenprothese im Bereich des auf dem Steigbügel-Köpfchen aufsitzenden, glockenförmigen zweiten Ankoppelelements mit erfindungsgemäß gestaltetem, halbkugeligen Auffütterungsabschnitt;
- Fig. 8b: wie in Fig. 8a, aber mit zweitem Ankoppelelement nach dem Stand der Technik; und
- Fig. 9: drei Aufnahmen mit jeweils gleicher Vergrößerung von unterschiedlichen, teilweise pathologisch veränderten menschlichen Steigbügel-Knöchelchen mit insbesondere verschiedenen Geometrien des jeweiligen Steigbügel-Köpfchens.

Die in den Figuren der Zeichnung schematisch dargestellten - im Detail unterschiedlich gestalteten - Ausführungsformen der erfindungsgemäßen **Gehörknöchelchenprothese 10a; 10b; 10c; 20a; 20b** weisen am einen Ende jeweils ein **erstes Ankoppelelement 11a; 11b; 11c; 21** auf, welches der mechanischen Verbindung der Prothese mit einem Glied der Gehörknöchelchenkette dient, als Trommelfell-Kopfplatte zur Anlage am Trommelfell oder als ein Anschlussstück 21 zur Schall leitenden Verbindung mit einem Aktor-Endstück eines aktiven Hör-Implantats gestaltet ist. Am anderen Ende der Gehörknöchelchenprothese 10a; 10b; 10c; 20a; 20b sitzt jeweils ein **zweites Ankoppelelement 12; 12'; 12"; 22; 82** mit einer **Zugangsöffnung 14; 24; 84** in einen **Aufnahmeraum 15; 25; 85,** das für eine mechanische Verbindung der Prothese mit dem **Steigbügel S**, insbesondere mit dem **Steigbügel**-**Köpfchen C**, als Glocke oder als Clip ausgebildet ist. Dazwischen ist ein die beiden Befestigungselemente 11; 21; 31; 41; 51 bzw. 12; 22; 32; 42; 52 Schall leitend miteinander verbindender **länglicher Prothesenkörper 13; 13a; 13b; 13c; 13d; 23** in Form eines länglichen Schaftes angeordnet.

Erfindungsgemäß ist das zweite Ankoppelelement 12; 12'; 12"; 22 jeweils geometrisch so gestaltet ist, dass es an der Innenfläche des Aufnahmeraums 15; 25 in axialer Fortsetzung des länglichen Prothesenkörpers 13; 13a; 13b; 13c; 13d; 23 einen vom länglichen Prothesenkörper 13; 13a; 13b; 13c; 13d; 23 weg in den Aufnahmeraum 15; 25 hinein ragenden **Auffütterungsabschnitt 16a; 16b; 16c; 16d; 16e; 16f; 16g; 16h; 16i; 26** aufweist, der im implantierten Zustand der Gehörknöchelchenprothese 10a; 10b; 10c; 20a; 20b am Steigbügel S, insbesondere am Steigbügel-Köpfchen C anliegt und die Ausbildung eines Hohlraums zwischen dem Steigbügel S und der Innenfläche des Aufnahmeraums 15; 25 in axialer Fortsetzung des länglichen Prothesenkörpers 13; 13a; 13b; 13c; 13d; 23 verhindert oder minimiert.

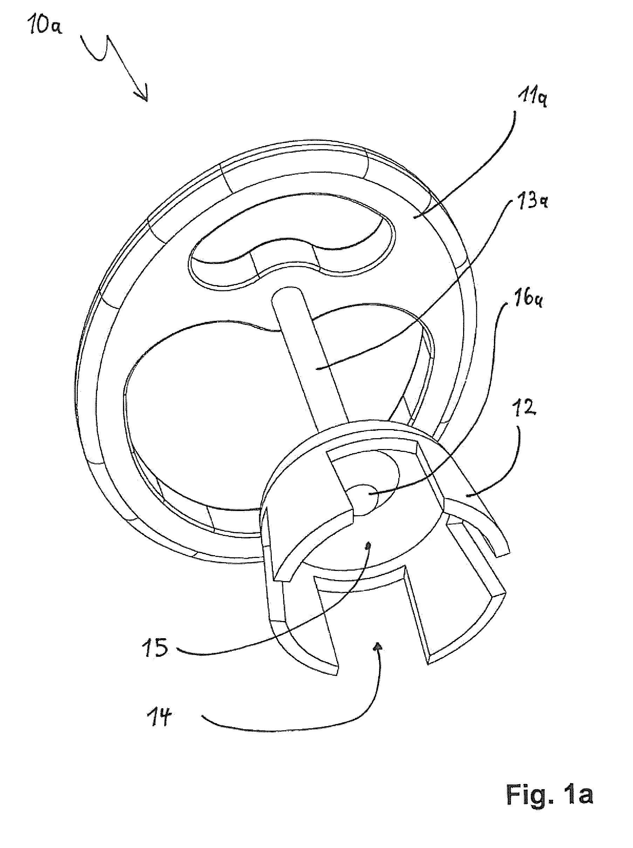

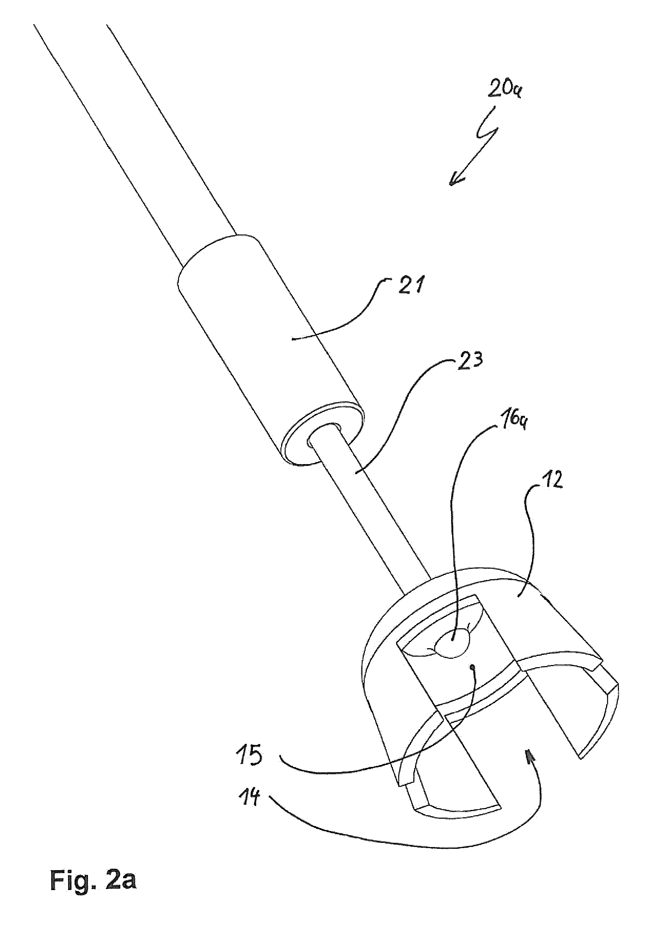

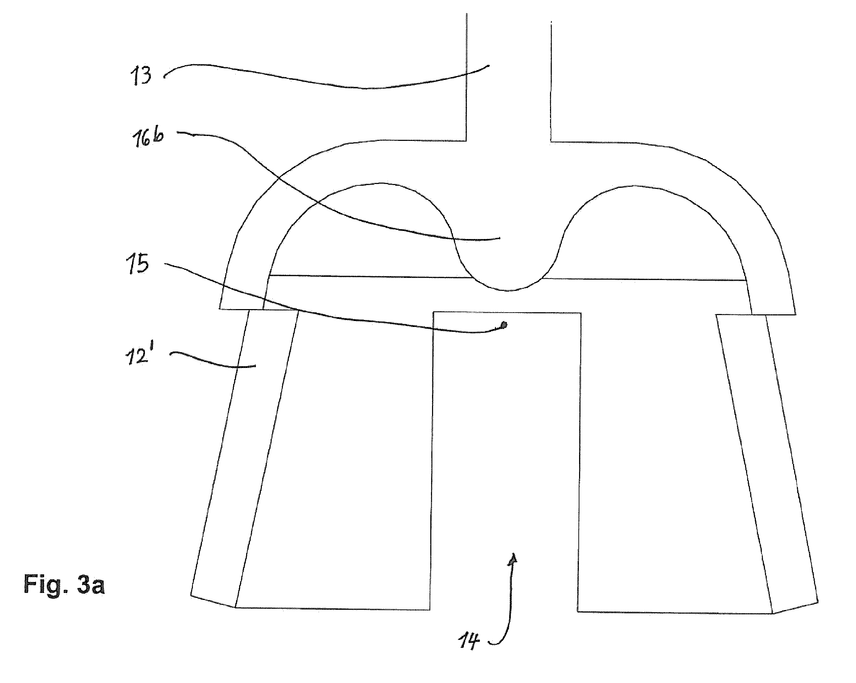

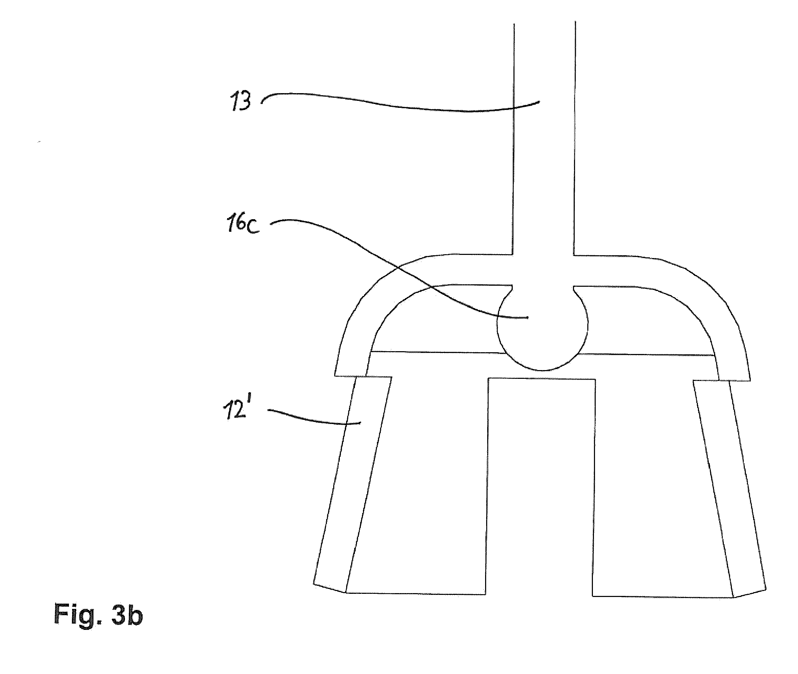

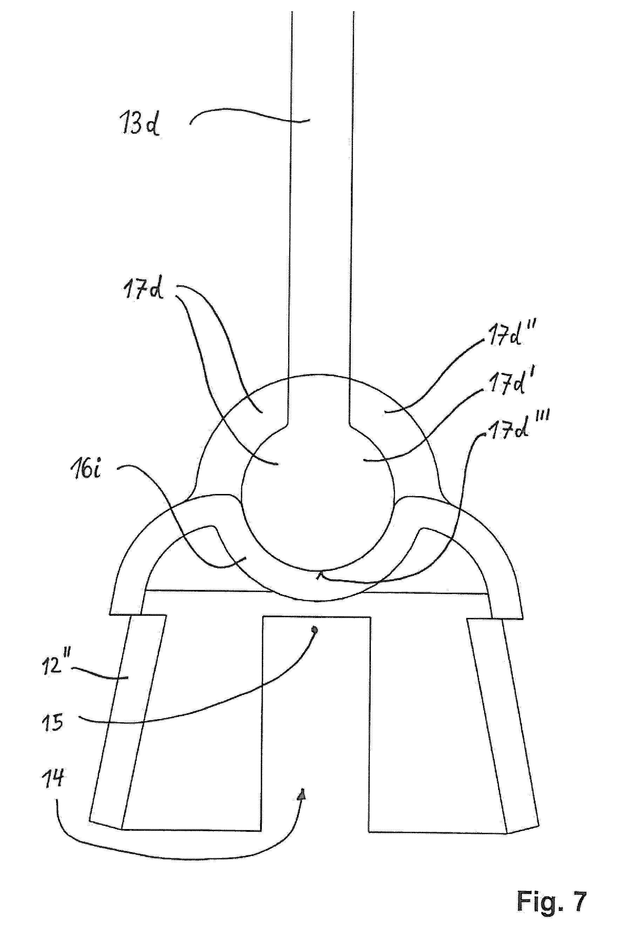

Bei den in den Figuren 1a bis 1c gezeigten Ausführungsformen ist das erste Ankoppelelement 11a; 11b; 11c in Form einer Kopfplatte zur Anlage am Trommelfell ausgebildet. Das zweite Ankoppelelement 12 an dem der Kopfplatte entgegen gesetzten Ende des länglichen Prothesenkörpers 13; 13a; 13b; 13c; 13d; 23 ist in diesen Ausführungsbeispielen als mehrfach geschlitzte Glocke zur Auflage auf dem Steigbügel-Köpfchen gestaltet und mit der Glockenhaube an einem Ende des länglichen Prothesenkörpers 13; 13a; 13b; 13c; 13d; 23 befestigt. Letzteres trifft auch für die in den Figuren 2a sowie 3a bis 8a dargestellten Ausführungsformen zu. Dabei weisen die glockenförmigen Ankoppelelemente 12 in den Figuren 1a bis 2a jeweils eine rund gewölbte Glockenhaube auf, das glockenförmige Ankoppelelement 12' in den Figuren 3a bis 8a eine abgeflachte und in Figur 7 eine oben eingedellte Glockenhaube.

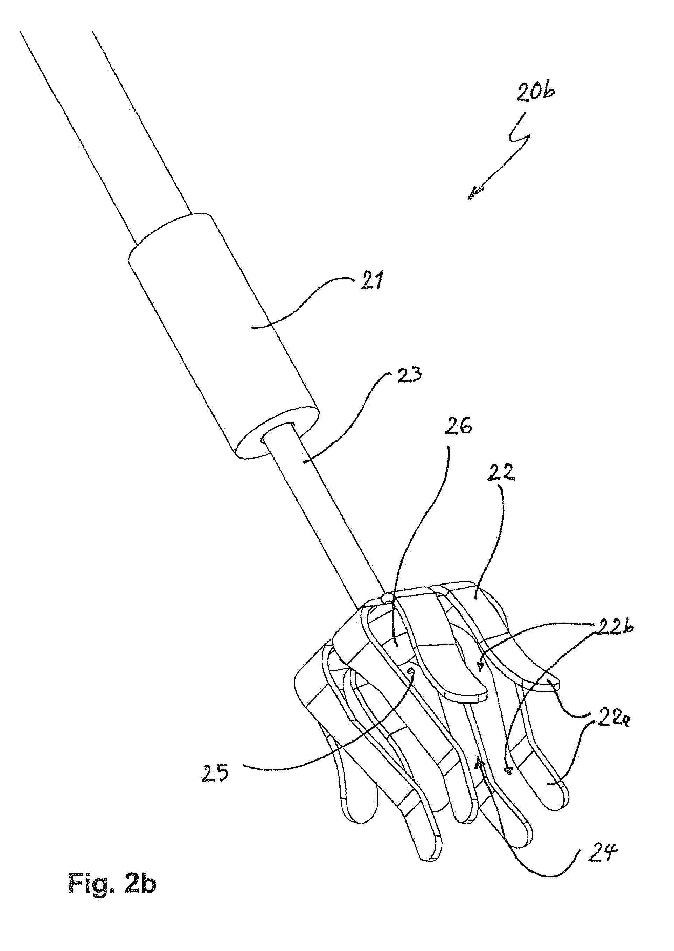

Bei den Ausführungsformen der Figuren 2 a und 2b ist das erste Ankoppelelement 21 als Anschlussstück zur Schall leitenden Verbindung mit einem Aktor-Endstück eines - in der Zeichnung nicht näher dargestellten - aktiven Hör-Implantats gestaltet. Das zweite Ankoppelelement 22 in Fig. 2b ist zudem als Clip mit mehreren, jeweils alternierenden seitlichen **Zungen 22a** und **Schlitzen 22b** ausgeführt. Der Auffütterungsabschnitt 16a; 16b; 16c; 16i; 26 bei den in den Ausführungsformen der Figuren 1a bis 3b sowie 7 und 8a ist kugelig oder ellipsoid gestaltet und symmetrisch zur verlängerten Achse des länglichen Prothesenkörpers 13; 13a; 13b; 13c; 13d; 23 angeordnet.

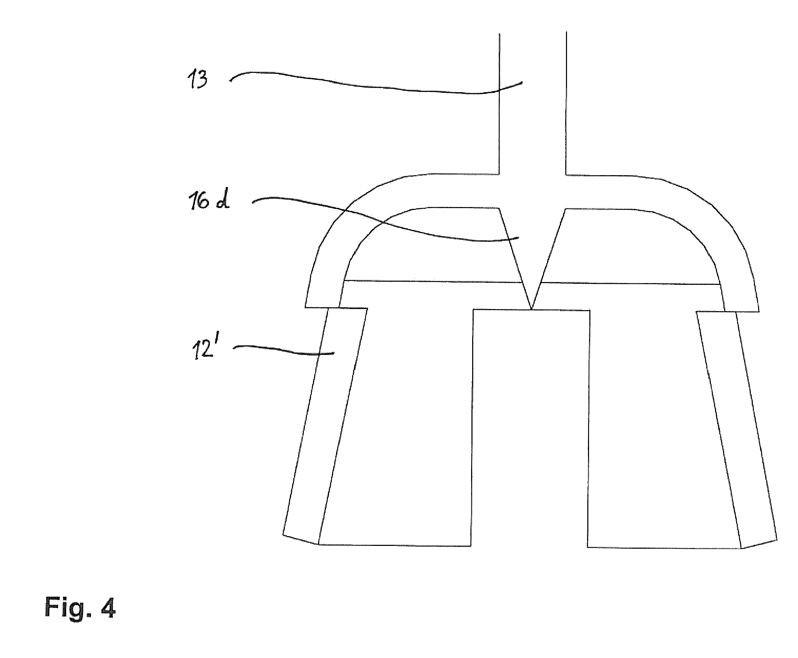

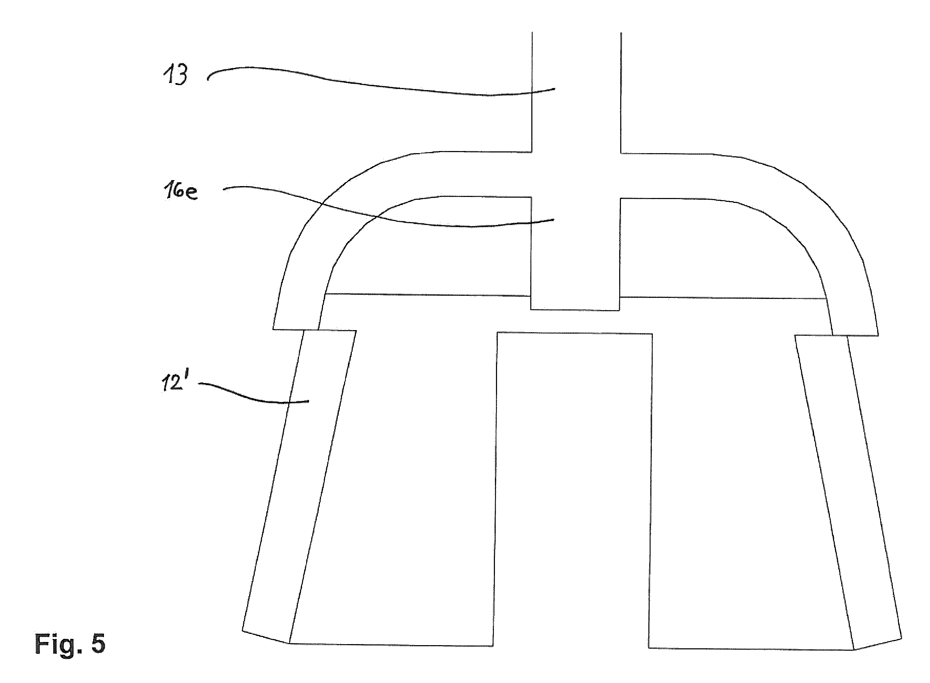

Bei der Fig. 4 gezeigten Ausführungsform ist der Auffütterungsabschnitt 16d kegelig gestaltet, während die das glockenförmige Ankoppelelement 12' in Fig. 5 einen zylinderförmigen Auffütterungsabschnitt 16e aufweist.

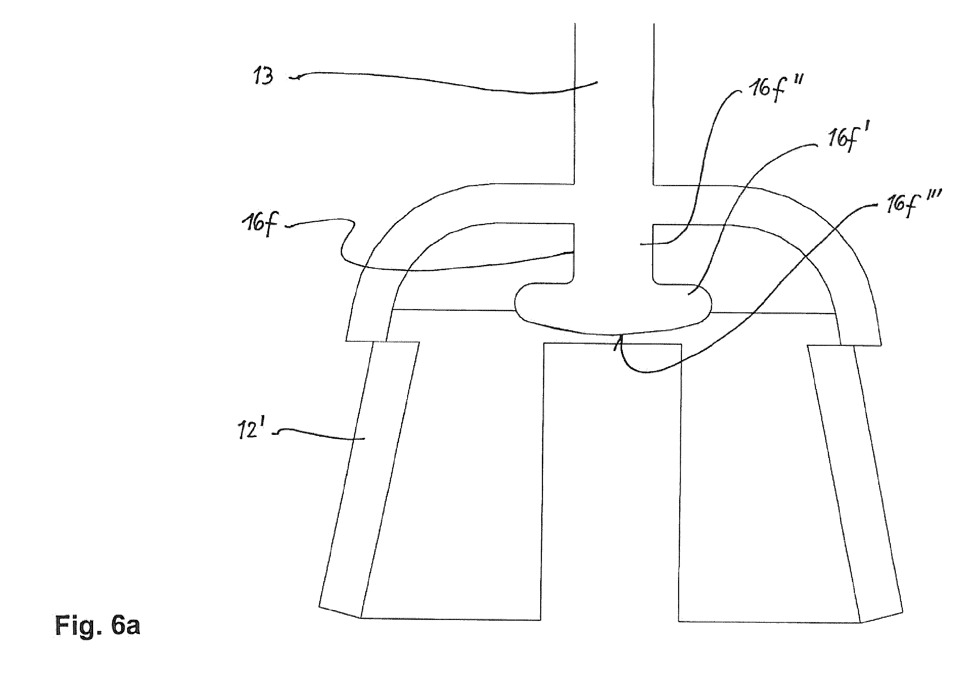

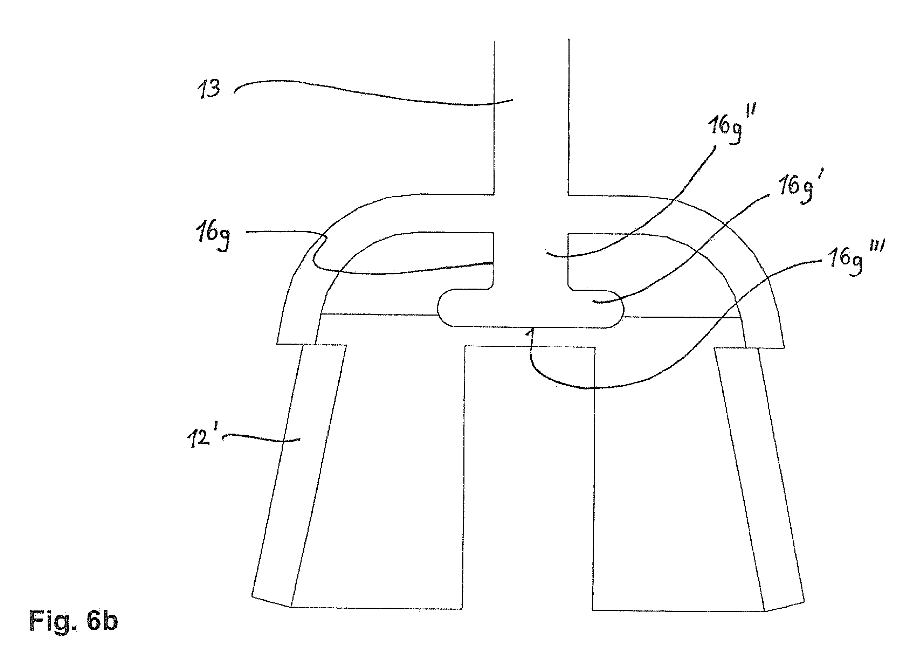

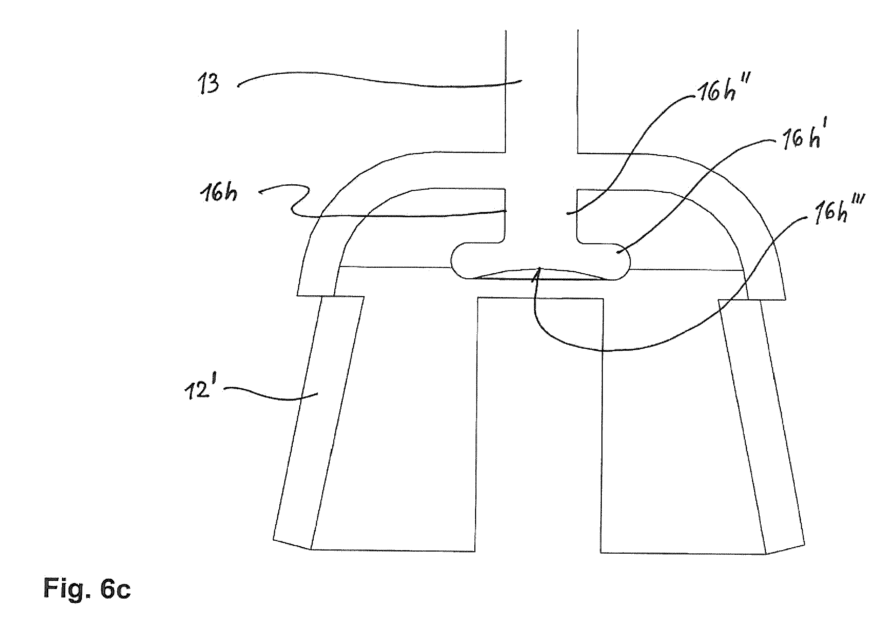

In den Figuren 6a bis 6c sind die Auffütterungsabschnitte 16f; 16g; 16h jeweils stempelförmig gestaltet und symmetrisch zur verlängerten Achse des länglichen Prothesenkörpers 13 angeordnet, wobei ein den **Stempelkörper 16f'; 16g'; 16h'** tragender **Stempelschaft 16f"; 16g"; 16h"** vom länglichen Prothesenkörpers 13 weg in den jeweiligen Aufnahmeraum 15 hinein ragt. In der Ausführungsform nach Fig. 6a weist der Stempelkörper 16f' eine in den Aufnahmeraum 15 gerichtete konkave Auflagefläche **16f"'** auf, in Fig. 6b eine ebene **Auflagefläche 16g"'** und in Fig. 6c **konvexe Auflagefläche 16h"'.**

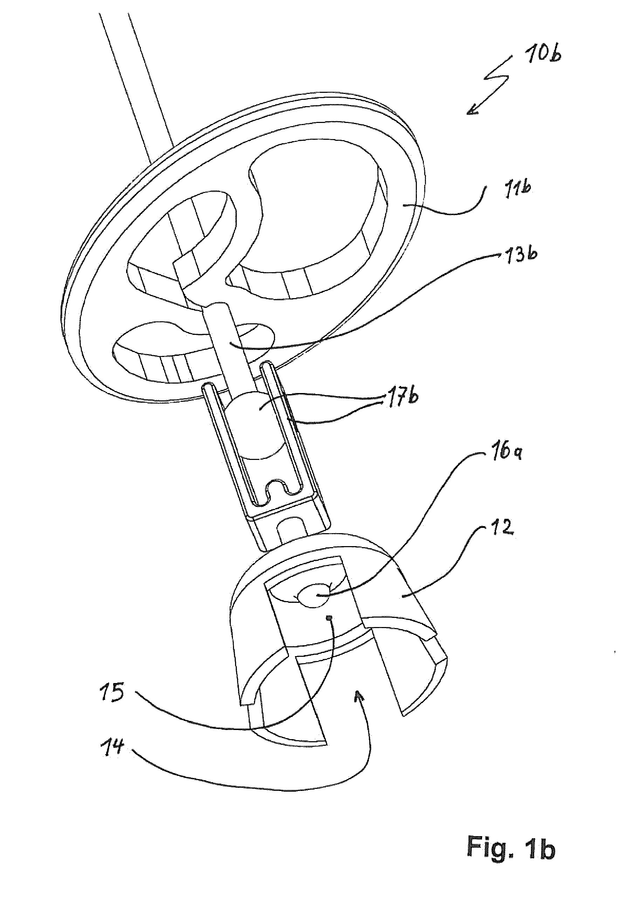

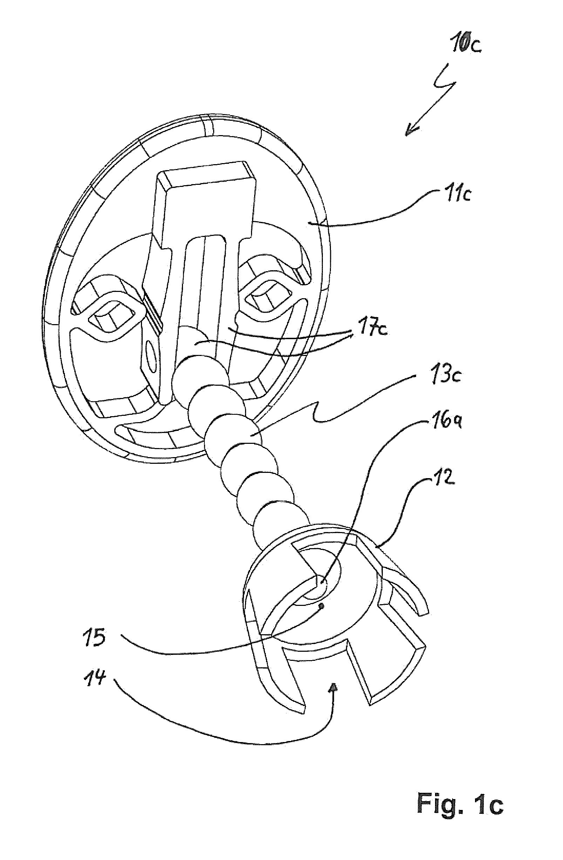

Die in den Figuren 1b, 1c und 7 dargestellten Ausführungsformen zeichnen sich dadurch aus, dass der längliche Prothesenkörper 13b; 13c; 13d mindestens ein Gelenk, insbesondere ein Kugelgelenk **17b; 17c; 17d** aufweist.

Während das Kugelgelenk 17b der Gehörknöchelchenprothese 10b in Fig. 1b in einen schaftförmigen längliche Prothesenkörper 13b integriert ist, weist die Gehörknöchelchenprothese 10c in Fig. 1c einen als Kugelkette ausgestalteten länglichen Prothesenkörper 13c auf. Dieser kann durch einen Aufnahmeabschnitt in dem als Trommelfell-Kopfplatte ausgeführten ersten Ankoppelelement 11c hindurchgeführt und auf die jeweils aktuell erforderliche Länge abgeschnitten werden. Die übrig bleibende oberste Kugel des länglichen Prothesenkörpers 13 bildet dann zusammen mit dem Aufnahmeabschnitt ein Kugelgelenk 17c.

Bei der Ausführungsform nach Fig. 7 umfasst das Kugelgelenk 17d eine an seinem dem zweiten Ankoppelelement 12" zugewandten Ende des länglichen Prothesenkörpers 13d angebrachte **Kugel 17d',** eine die Kugel 17d' auf ihrer vom zweiten Ankoppelelement 12" abgewandten Seite bedeckende **Hülle 17d"** sowie eine in die Außenfläche der Glockenhaube des zweiten Ankoppelelements 12" eingedellte **Vertiefung 17d"',** welche für die Kugel 17d' als Gelenkpfanne wirkt. Die Hülle 17d" ist aus einem Kunststoff-Verguss, vorzugsweise aus einem Silikon-Verguss gebildet. Die dem Aufnahmeraum 15 zugewandte Innenseite der Glockenhaube bildet aufgrund der oben beschriebenen Eindellung einen halbkugeligen Auffütterungsabschnitt 16i.

In Fig. 8a ist eine erfindungsgemäß in ihrem glockenförmigen zweiten Ankoppelelement 12' mit einem Auffütterungsabschnitt 16b modifizierte Prothese einer in Fig. 8b dargestellten Gehörknöchelchenprothese nach dem Stand der Technik optisch gegenübergestellt. Deutlich ist zu erkennen, dass bei der herkömmlichen Prothese in Fig. 8b gerade zwischen der axialen Verlängerung des länglichen Prothesenkörpers 83 und dem Steigbügel-Köpfchen C zwangsläufig ein Hohlraum freibleibt, welcher sich auf die Schallleitung in Richtung Innenohr äußerst negativ auswirkt. Demgegenüber wird ebendieser Hohlraum in Verlängerung des länglichen Prothesenkörpers 13 bei der erfindungsgemäßen Prothese in Fig. 8a durch den Auffütterungsabschnitt 16b sicher vermieden.

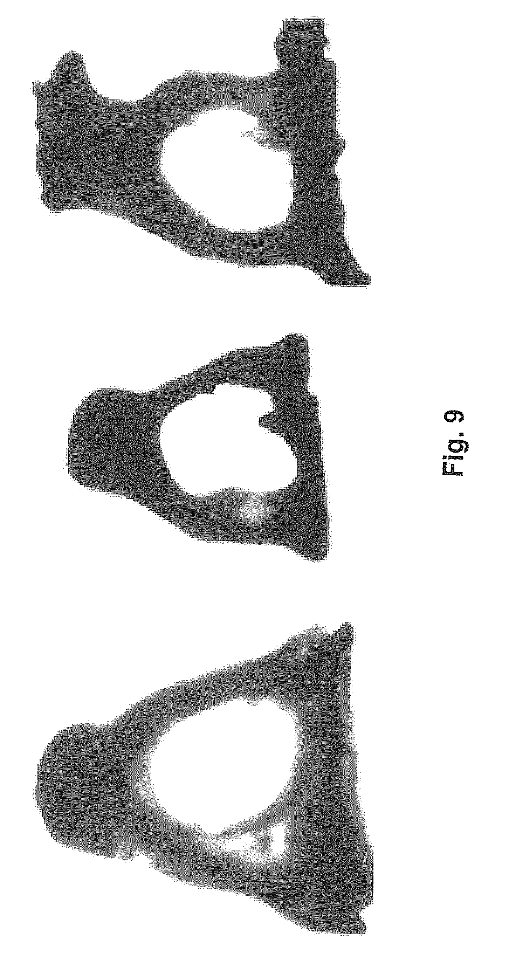

In Fig. 9 schließlich sind drei photographische Aufnahmen von menschlichen Steigbügel-Knöchelchen mit jeweils gleicher Vergrößerung gezeigt. Man erkennt erhebliche Unterschiede in der geometrischen Form des Steigbügels, insbesondere im Bereich des Steigbügel-Köpfchens, aber auch deutlich verschiedene Größen des jeweiligen Steigbügel-Knöchelchens sowie seiner Details, die teilweise durch pathologische Prozesse oder einfach nur durch die natürlichen individuellen Abweichung von einem Patienten zum anderen hervorgerufen werden. Hier sind die entscheidenden Vorteile verdeutlicht, welche die Variationsmöglichkeiten bei einer erfindungsgemäß modifizierten Gehörknöchelchenprothese im Gegensatz zu einer herkömmlichen Prothese nach dem Stand der Technik im Hinblick auf eine individuelle Problemlösung bieten. Aufgrund der diversen Ausgestaltungsmöglichkeiten des erfindungsgemäß vorgesehenen Auffütterungsabschnitts kann gerade im Bereich des Steigbügel-Köpfchens eine optimale Schall-Weiterleitung sichergestellt werden.

## Patentansprüche

1. Gehörknöchelchenprothese (10a; 10b; 10c; 20a; 20b), die zum Ersatz oder Überbrücken mindestens eines Elements der menschlichen Gehörknöchelchenkette ausgebildet ist, mit einem Schall übertragenden länglichen Prothesenkörper (13; 13a; 13b; 13c; 13d; 23), welcher an seinem einen Ende ein erstes Ankoppelelement (11a; 11b; 11c; 21) aufweist, das als eine Kopfplatte (11a; 11b; 11c) zur Anlage der Prothese am Trommelfell oder als ein Clip zur mechanischen Verbindung mit einem Glied der Gehörknöchelchenkette oder als ein Anschlussstück (21) zur Schall leitenden Verbindung mit einem Aktor-Endstück eines aktiven Hör-Implantats gestaltet ist, wobei der längliche Prothesenkörper (13; 13a; 13b; 13c; 13d; 23) an seinem anderen Ende ein zweites Ankoppelelement (12; 12'; 12"; 22) mit einer Zugangsöffnung (14; 24) in einen Aufnahmeraum (15; 25) aufweist, das für eine mechanische Verbindung der Prothese mit einem Steigbügel oder mit einem Steigbügel-Köpfchen, als Glocke (12; 12'; 12") oder als Clip (22) ausgebildet ist,
**dadurch gekennzeichnet,**
**dass** das zweite Ankoppelelement (12; 12'; 12"; 22) an der Innenfläche des Aufnahmeraums (15; 25) in axialer Fortsetzung des länglichen Prothesenkörpers (13; 13a; 13b; 13c; 13d; 23) einen vom länglichen Prothesenkörper (13; 13a; 13b; 13c; 13d; 23) weg in den Aufnahmeraum (15; 25) hinein ragenden Auffütterungsabschnitt (16a; 16b; 16c; 16d; 16e; 16f; 16g; 16h; 16i; 26) aufweist, der im implantierten Zustand der Gehörknöchelchenprothese (10a; 10b; 10c; 20a; 20b) am Steigbügel, insbesondere am Steigbügel-Köpfchen anliegt und die Ausbildung eines Hohlraums zwischen dem Steigbügel und der Innenfläche des Aufnahmeraums (15; 25) in axialer Fortsetzung des länglichen Prothesenkörpers (13; 13a; 13b; 13c; 13d; 23) verhindert oder minimiert.

2. Gehörknöchelchenprothese nach Anspruch 1, **dadurch gekennzeichnet, dass** das zweite Ankoppelelement als seitlich mehrfach geschlitzte Glocke (12; 12'; 12") ausgeführt und mit einer Glockenhaube an einem Ende des länglichen Prothesenkörpers (13; 13a; 13b; 13c; 13d) befestigt ist.

3. Gehörknöchelchenprothese nach Anspruch 2, **dadurch gekennzeichnet, dass** die Glocke (12) eine rund gewölbte Glockenhaube aufweist.

4. Gehörknöchelchenprothese nach Anspruch 2, **dadurch gekennzeichnet, dass** die Glocke (12'; 12") eine abgeflachte und/oder von oben eingedellte Glockenhaube aufweist.

5. Gehörknöchelchenprothese nach Anspruch 1, **dadurch gekennzeichnet, dass** das zweite Ankoppelelement als Clip (22) mit mehreren, jeweils alternierenden seitlichen Zungen (22a) und Schlitzen (22b) ausgeführt ist.

6. Gehörknöchelchenprothese nach einem Ansprüche 1 bis 5, **dadurch gekennzeichnet, dass** der Auffütterungsabschnitt (16a; 16b; 16c; 16i; 26) kugelig oder ellipsoid gestaltet und symmetrisch zur verlängerten Achse des länglichen Prothesenkörpers (13; 13a; 13b; 13c; 13d; 23) angeordnet ist.

7. Gehörknöchelchenprothese nach einem Ansprüche 1 bis 5, **dadurch gekennzeichnet, dass** der Auffütterungsabschnitt (16d) kegelförmig gestaltet und symmetrisch zur verlängerten Achse des länglichen Prothesenkörpers (13) angeordnet ist, wobei eine Kegelspitze vom länglichen Prothesenkörpers (13) weg in den Aufnahmeraum (15) hinein ragt.

8. Gehörknöchelchenprothese nach einem Ansprüche 1 bis 5, **dadurch gekennzeichnet, dass** der Auffütterungsabschnitt (16e) zylinderförmig gestaltet und symmetrisch zur verlängerten Achse des länglichen Prothesenkörpers (13) angeordnet ist, wobei ein Zylinder vom länglichen Prothesenkörpers (13) weg in den Aufnahmeraum (15) hinein ragt.

9. Gehörknöchelchenprothese nach einem Ansprüche 1 bis 5, **dadurch gekennzeichnet, dass** der Auffütterungsabschnitt (16f; 16g; 16h) stempelförmig gestaltet und symmetrisch zur verlängerten Achse des länglichen Prothesenkörpers (13) angeordnet ist, wobei ein einen Stempelkörper (16f'; 16g'; 16h') tragender Stempelschaft (16f"; 16g"; 16h") vom länglichen Prothesenkörpers (13) weg in den Aufnahmeraum (15) hinein ragt.

10. Gehörknöchelchenprothese nach Anspruch 9, **dadurch gekennzeichnet, dass** der Stempelkörper (16f') eine in den Aufnahmeraum (15) gerichtete konkave Auflagefläche (16f") aufweist.

11. Gehörknöchelchenprothese nach Anspruch 9, **dadurch gekennzeichnet, dass** der Stempelkörper (16g') eine in den Aufnahmeraum (15) gerichtete ebene Auflagefläche (16g"') aufweist.

12. Gehörknöchelchenprothese nach Anspruch 9, **dadurch gekennzeichnet, dass** der Stempelkörper (16h') eine in den Aufnahmeraum (15) gerichtete konvexe Auflagefläche (16h"') aufweist.

13. Gehörknöchelchenprothese nach einem der vorhergehenden Ansprüche, **dadurch gekennzeichnet, dass** der längliche Prothesenkörper (13b; 13c; 13d) mindestens ein Gelenk (17b; 17c; 17d) aufweist.

14. Gehörknöchelchenprothese nach Anspruch 13, **dadurch gekennzeichnet, dass** das Gelenk (17d) eine an seinem dem zweiten Ankoppelelement (12") zugewandten Ende des länglichen Prothesenkörpers (13d) angebrachte Kugel (17d'), eine die Kugel (17d') auf ihrer vom zweiten Ankoppelelement (12") abgewandten Seite bedeckende Hülle (17d") sowie eine Vertiefung (17d"') in der dem zweiten Ankoppelelement (12") zugewandten Seite umfasst, welche für die Kugel (17d') als Gelenkpfanne wirkt.

15. Gehörknöchelchenprothese nach Anspruch 14, **dadurch gekennzeichnet, dass** die Hülle (17d") aus einem Kunststoff-Verguss gebildet ist.

## Claims

1. An ossicular prosthesis (10a; 10b; 10c; 20a; 20b), designed to replace or bridge at least one element of the human ossicular chain, said prosthesis comprising an elongate, sound-transmitting prosthesis body (13; 13a; 13b; 13c; 13d; 23) with a first coupling element (11a; 11b; 11c; 21) at one end formed as a headplate (11a; 11b; 11c) for placement of the prosthesis against the tympanic membrane, or as a clip for mechanical connection to an element of the ossicular chain, or as a connecting piece (21) for a sound-conducting connection to an actuator end piece of an active auditory implant, the elongate body of the prosthesis (13; 13a; 13b; 13c; 13d; 23) having a second coupling element (12; 12'; 12"; 22) at its other end which has an access opening (14; 24) into a receiving space (15; 25) and takes the form of a bell (12; 12'; 12") or a clip (22) for mechanical connection of the prosthesis to a stapes or to the head of a stapes,
**characterized in that**
the second coupling element (12; 12'; 12"; 22) has, on the inner face of the receiving space (15; 25) in axial continuation of the elongate prosthesis body (13; 13a; 13b; 13c; 13d; 23), a fill-in piece (16a; 16b; 16c; 16d; 16e; 16f; 16g; 16h; 16i; 26) projecting from the elongate prosthesis body (13; 13a; 13b; 13c; 13d; 23) into the receiving space (15; 25), said fill-in piece (16a; 16b; 16c; 16d; 16e; 16f; 16g; 16h; 16i; 26) resting against the stapes, specifically against the head of the stapes, in the implanted state of the ossicular prosthesis (10a; 10b; 10c; 20a; 20b) and preventing or minimizing the formation of a cavity between the stapes and the inner face of the receiving space (15; 25) in axial continuation of the elongate prosthesis body (13; 13a; 13b; 13c; 13d; 23).

2. An ossicular prosthesis according to Claim 1, **characterized in that** the second coupling element takes the form of a bell (12; 12'; 12") having a number of lateral slots, this being attached by means of a bell cap to one end of the elongate prosthesis body (13; 13a; 13b; 13c; 13d).

3. An ossicular prosthesis according to Claim 2, **characterized in that** the bell (12) has a roundly curved bell cap.

4. An ossicular prosthesis according to Claim 2, **characterized in that** the bell (12'; 12") has a bell cap which is flattened and/or is indented from above.

5. An ossicular prosthesis according to Claim 1, **characterized in that** the second coupling element takes the form of a clip (22) having a plurality of alternating lateral prongs (22a) and slots (22b).

6. An ossicular prosthesis according to one of Claims 1 to 5, **characterized in that** the fill-in piece (16a; 16b; 16c; 16i; 26) is globular or ellipsoidal in shape and is disposed so as to be symmetrical relative to the extended axis of the elongate prosthesis body (13; 13a; 13b; 13c; 13d; 23).

7. An ossicular prosthesis according to one of Claims 1 to 5, **characterized in that** the fill-in piece (16d) is conical in form and is disposed so as to be symmetrical relative to the extended axis of the elongate prosthesis body (13), the apex of the cone projecting from the elongate prosthesis body (13) into the receiving space (15).

8. An ossicular prosthesis according to one of Claims 1 to 5, **characterized in that** the fill-in piece (16e) is cylindrical in form and is disposed so as to be symmetrical relative to the extended axis of the prosthesis body (13), a cylinder projecting from the elongate prosthesis body (13) into the receiving space (15).

9. An ossicular prosthesis according to one of Claims 1 to 5, **characterized in that** the fill-in piece (16f; 16g; 16h) is plunger-shaped and is disposed so as to be symmetrical relative to the extended axis of the elongate prosthesis body (13), a shaft (16f"; 16g"; 16h") bearing the plunger body (16f; 16g'; 16h') projecting from the elongate prosthesis body (13) into the receiving space (15).

10. An ossicular prosthesis according to Claim 9, **characterized in that** the plunger body (16f) has a concave bearing surface (16f") directed into the receiving space (15).

11. An ossicular prosthesis according to Claim 9, **characterized in that** the plunger body (16g') has a flat bearing surface (16g"') directed into the receiving space (15).

12. An ossicular prosthesis according to Claim 9, **characterized in that** the plunger body (16h') has a convex bearing surface (16h"') directed into the receiving space (15).

13. An ossicular prosthesis according to one of the preceding claims, **characterized in that** the elongate prosthesis body (13b; 13c; 13d) has at least one joint (17b; 17c; 17d).

14. An ossicular prosthesis according to Claim 13, **characterized in that** the joint (17d) comprises a ball (17d') provided on the end of the elongate prosthesis body (13d) facing the second coupling element (12"), a casing (17d") covering the ball (17d') on its side remote from the second coupling element (12") and a recess (17d"') which is located in the side facing the coupling element (12") and serves as a socket for the ball (17d').

15. An ossicular prosthesis according to Claim 14, **characterized in that** the casing (17d") is made of a plastic casting compound.

## Revendications

1. Prothèse pour les osselets de l'oreille (10a ; 10b ; 10c ; 20a ; 20b), qui est constituée pour remplacer ou ponter au moins un élément de la chaîne des osselets de l'oreille humaine, avec un corps de prothèse (13 ; 13a ; 13b ; 13c ; 13d ; 23) allongé transmettant le son qui présente à une extrémité un premier élément d'accouplement (11 a ; 11 b ; 11 c ; 21) qui est constitué en tant que plaque de tête (11a ; 11b ; 11c) pour l'appui de la prothèse sur le tympan ou en tant que clip pour le raccordement mécanique avec un organe de la chaîne des osselets de l'oreille ou en tant que pièce de connexion (21) pour le raccordement conduisant le son avec une pièce finale d'actionneur d'un implant auditif actif, dans laquelle le corps de prothèse (13 ; 13a ; 13b ; 13c ; 13d ; 23) allongé présente à son autre extrémité un deuxième élément d'accouplement (12 ; 12' ; 12" ; 22) qui est doté d'une ouverture d'accès (14 ; 24) vers un espace de réception (15 ; 25) et qui est constitué en tant que cloche (12 ; 12' ; 12") ou en tant que clip (22) pour un raccordement mécanique de la prothèse avec un étrier ou avec une petite tête d'étrier,
**caractérisée en ce que**
le deuxième élément d'accouplement (12; 12'; 12"; 22) présente, sur la face intérieure de l'espace de réception (15; 25), dans le prolongement axial du corps de prothèse (13 ; 13a ; 13b ; 13c ; 13d ; 23) allongé, un segment de chemisage (16a ; 16b ; 16c ; 16d ; 16e; 16f; 16g ; 16h, 16i ; 26) qui dépasse du corps de prothèse (13 ; 13a ; 13b ; 13c ; 13d ; 23) allongé dans l'intérieur de l'espace de réception (15 ; 25) et qui, dans l'état implanté de la prothèse pour les osselets de l'oreille (10a ; 10b; 10c; 20a; 20b), est adjacent à l'étrier, en particulier à la petite tête d'étrier, et empêche ou minimise la formation d'une cavité entre l'étrier et la face intérieure de l'espace de réception (15 ; 25) dans le prolongement axial du corps de prothèse (13 ; 13a ; 13b ; 13c ; 13d ; 23) allongé.

2. Prothèse pour les osselets de l'oreille selon la revendication 1, **caractérisée en ce que** le deuxième élément d'accouplement est réalisé en tant que cloche (12, 12', 12") à multiples fentes latérales et est fixé avec un capot de cloche à une extrémité du corps de prothèse (13 ; 13a ; 13b ; 13c ; 13d) allongé.

3. Prothèse pour les osselets de l'oreille selon la revendication 2, **caractérisée en ce que** la cloche (12) présente un capot de cloche bombé de façon arrondie.

4. Prothèse pour les osselets de l'oreille selon la revendication 2, **caractérisée en ce que** la cloche (12' ; 12 ") présente un capot de cloche aplati et/ou enfoncé à partir du haut.

5. Prothèse pour les osselets de l'oreille selon la revendication 1, **caractérisée en ce que** le deuxième élément d'accouplement est réalisé en tant que clip (22) avec plusieurs languettes (22a) et fentes (22b) latérales respectivement alternées.

6. Prothèse pour les osselets de l'oreille selon l'une des revendications 1 à 5, **caractérisée en ce que** le segment de chemisage (16a ; 16b ; 16c ; 16i ; 26) est constitué à la façon d'une sphère ou d'un ellipsoïde et est disposé de façon symétrique par rapport à l'axe prolongé du corps de prothèse (13 ; 13a ; 13b ; 13c ; 13d ; 23) allongé.

7. Prothèse pour les osselets de l'oreille selon l'une des revendications 1 à 5, **caractérisée en ce que** le segment de chemisage (16d) est constitué de façon conique et disposé de façon symétrique par rapport à l'axe prolongé du corps de prothèse (13) allongé, une pointe conique dépassant du corps de prothèse (13) allongé vers l'intérieur de l'espace de réception (15).

8. Prothèse pour les osselets de l'oreille selon l'une des revendications 1 à 5, **caractérisée en ce que** le segment de chemisage (16e) est constitué de façon cylindrique et disposé de façon symétrique par rapport à l'axe prolongé du corps de prothèse (13) allongé, un cylindre dépassant du corps de prothèse (13) allongé vers l'intérieur de l'espace de réception (15)

9. Prothèse pour les osselets de l'oreille selon l'une des revendications 1 à 5, **caractérisée en ce que** le segment de chemisage (16f ; 16g ; 16h) est constitué en forme de poinçon et disposé de façon symétrique par rapport à l'axe prolongé du corps de prothèse (13) allongé, une tige de poinçon (16f", 16g" ; 16h") qui porte un corps de poinçon (16f' ; 16g'; 16h') dépassant du corps de prothèse (13) allongé vers l'intérieur de l'espace de réception (15).

10. Prothèse pour les osselets de l'oreille selon la revendication 9, **caractérisée en ce que** le corps de poinçon (16f') présente une face d'appui (16f"') concave orientée vers l'intérieur de l'espace de réception (15).

11. Prothèse pour les osselets de l'oreille selon la revendication 9, **caractérisée en ce que** le corps de poinçon (16g') présente une face d'appui (16g"') plane orientée vers l'intérieur de l'espace de réception (15).

12. Prothèse pour les osselets de l'oreille selon la revendication 9, **caractérisée en ce que** le corps de poinçon (16h') présente une face d'appui (16h"') convexe orientée vers l'intérieur de l'espace de réception (15).

13. Prothèse pour les osselets de l'oreille selon l'une des revendications précédentes, **caractérisée en ce que** le corps de prothèse (13b ; 13c ; 13d) allongé présente au moins une articulation (17b ; 17c ; 17d).

14. Prothèse pour les osselets de l'oreille selon la revendication 13, **caractérisée en ce que** l'articulation (17d) comprend, à son extrémité du corps de prothèse (13d) allongé tournée vers le deuxième élément d'accouplement (12"), une sphère (17d') mise en place, une enveloppe (17d") couvrant la sphère (17d') sur son côté opposé au deuxième élément d'accouplement (12") ainsi qu'un creux (17d"') dans le côté tourné vers le deuxième élément d'accouplement (12") qui agit en tant que cavité articulaire pour la sphère (17d').

15. Prothèse pour les osselets de l'oreille selon la revendication 14, **caractérisée en ce que** l'enveloppe (17d") est formée d'un enrobage en matière plastique.
